# FASCICULE DE BREVET EUROPEEN

(11) **EP 1 407 800 B1**
(45) Date de publication et mention de la délivrance du brevet: **05.03.2008**
(21) Numéro de dépôt: 03292054.8
(22) Date de dépôt: 20.08.2003
(51) Int. Cl.: A61N 1/37

(54) **Dispositif médical implantable actif tel que stimulateur cardiaque double chambre, pourvu de moyens perfectionnés d'ajustement de la sensibilité auriculaire et de l'énergie de stimulation auriculaire**
Implantierbare medizinische aktive Vorrichtung wie z. B. Zweikammer-Herzschrittmacher mit verbesserter Justierung der Vorhofsempfindlichkeit und des Vorhofstimulationsenergiepegels
Implantable medical active device, like dual chamber pacing, with improved adjustment of the atrial sensitivity and of the energy level of the atrial pacing

(30) Priorité: 21.08.2002 FR 0210458
(43) Date de publication de la demande: 14.04.2004
(73) Titulaire: ELA MEDICAL, 92541 Montrouge (FR)
(72) Inventeur: Amblard, Amel, 92290 Chatenay Malabry (FR)
(74) Mandataire: Freneaux, Julien

(56) Documents cités:
- EP-B- 0 550 342
- US-B1- 6 339 723

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, dispositifs "multisite" (triple ou quadruple chambre), défibrillateurs et/ou cardioverteurs, permettant de délivrer au coeur des impulsions électriques de faible énergie pour le traitement des troubles du rythme cardiaque.

Elle concerne ceux de ces dispositifs qui comprennent des circuits de stimulation double chambre.

Le US-A-6 339 723 décrit un tel dispositif, qui comprend des moyens permettant de s'assurer que la capture auriculaire n'est pas faussée par la survenue de fusions, c'est-à-dire de stimulations intervenant de façon concomitante à une dépolarisation auriculaire spontanée.

L'invention concerne plus particulièrement les dispositifs double-chambre qui sont capables d'une commutation automatique de mode de fonctionnement (CAM), comme par exemple ceux décrits par les EP-A-0 488 904 (ELA Médical) et EP-A-1 048 322 (ELA Médical).

La qualité de la détection des signaux cardiaques et la qualité de la capture sont un pré-requis indispensable à l'efficacité des différents algorithmes d'analyse et de pilotage intégrés au dispositif. Des paramètres sont disponibles à cet effet pour permettre au médecin d'ajuster au mieux la sensibilité de détection et l'énergie de stimulation. Néanmoins, les valeurs optimales sont susceptibles d'évoluer en fonction de circonstances cliniques ou mécaniques (microdéplacement de sonde ou effet de médicaments par exemple), ces variations pouvant être paroxystiques ou permanentes.

L'invention propose de remédier à ces difficultés en apportant un perfectionnement aux dispositifs connus.

Plus précisément, l'un des buts de l'invention est de proposer des moyens perfectionnés d'auto-ajustage des valeurs de sensibilité et d'énergie de stimulation, propres à éviter des déréglages intempestifs et inutiles de ces paramètres.

Essentiellement, l'invention propose de détecter les situations de sous-détection auriculaire ou de perte de capture auriculaire, pour pouvoir garantir le bon fonctionnement des différents algorithmes équipant le dispositif.

La détection et la correction des défauts de capture ou de sous-détection auriculaires permet d'éviter notamment un basculement inapproprié en mode DDD, évitant de stimuler le ventricule inutilement et palliant ainsi les possibles effets délétères, du point de vue hémodynamique, d'une telle situation inappropriée.

Le type de dispositif auquel s'applique l'invention est un dispositif double chambre tel que décrit par exemple dans le EP-A-0 488 904 précité, et correspondant au préambule de la revendication 1. Selon l'invention, il comprend les éléments énoncés dans la partie caractérisante de cette revendication 1. Les sous-revendications visent des formes de mise en oeuvre particulières avantageuses.

On va maintenant décrire un exemple de mise en oeuvre de l'invention, en référence aux dessins annexés.

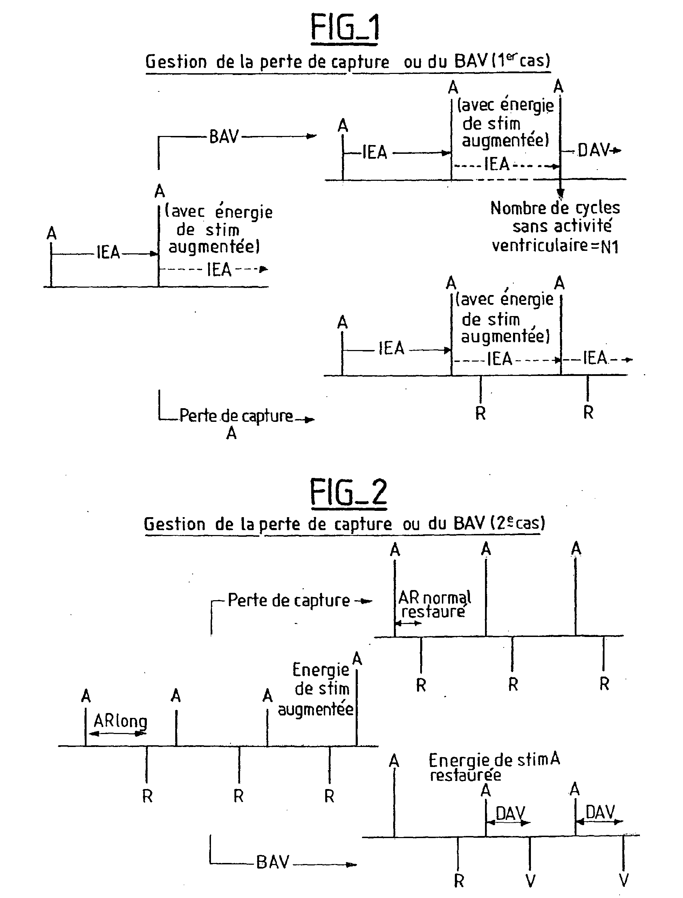

Les figures 1 à 5 sont des chronogrammes correspondant aux dives modes de fonctionnement du dispositif de l'invention

L'invention peut être mise en oeuvre par une programmation appropriée du logiciel de commande d'un stimulateur connu, ce logiciel intégrant par exemple un algorithme du type DDD-CAM tel que celui décrit par les EP-A-0 488 904 et EP-A-1 048 322 précités.

On va tout d'abord donner un certain nombre de définitions utilisées dans la suite de la description.

*Détection P :* recueil d'une activité spontanée ayant son origine dans l'oreillette ; on considérera qu'il y a effectivement détection P si celle-ci n'est pas suivie dans un délai donné, par exemple dans les 31 ms, par une détection ventriculaire (sinon, on se trouverait dans une situation de *"far-field* ventriculaire", c'est-à-dire de recueil via l'oreillette d'une dépolarisation lointaine provenant du ventricule).

*Détection R* : recueil d'une activité spontanée ayant son origine dans le ventricule.

*Stimulation A* : stimulation délivrée à l'oreillette.

*Stimulation V* : stimulation délivrée au ventricule.

*Événement auriculaire* : détection P ou bien stimulation A ;

*Événement ventriculaire* : détection R ou bien stimulation V ;

*Cycle cardiaque :* intervalle de temps séparant deux événements de même nature dans la même cavité, par exemple séparant deux détections P, ou deux stimulations A.

*PP moyen :* intervalle moyen du rythme auriculaire, calculé par exemple sur huit cycles cardiaques ne comprenant pas d'extrasystole.

*Intervalle d'échappement (lE) :* intervalle de temps, compté après une détection ou une stimulation dans une cavité donnée, à l'issue duquel une stimulation est délivrée à cette cavité si aucun événement spontané n'a été détecté dans cette même cavité. Pour l'oreillette, il s'agit de l'intervalle d'échappement auriculaire (IEA).

*Extrasystole auriculaire (ESA) :* détection auriculaire survenant à l'intérieur de la période réfractaire auriculaire post-auriculaire (PRAPA), le calcul de cette PRAPA étant celui d'un stimulateur de type DDD standard.

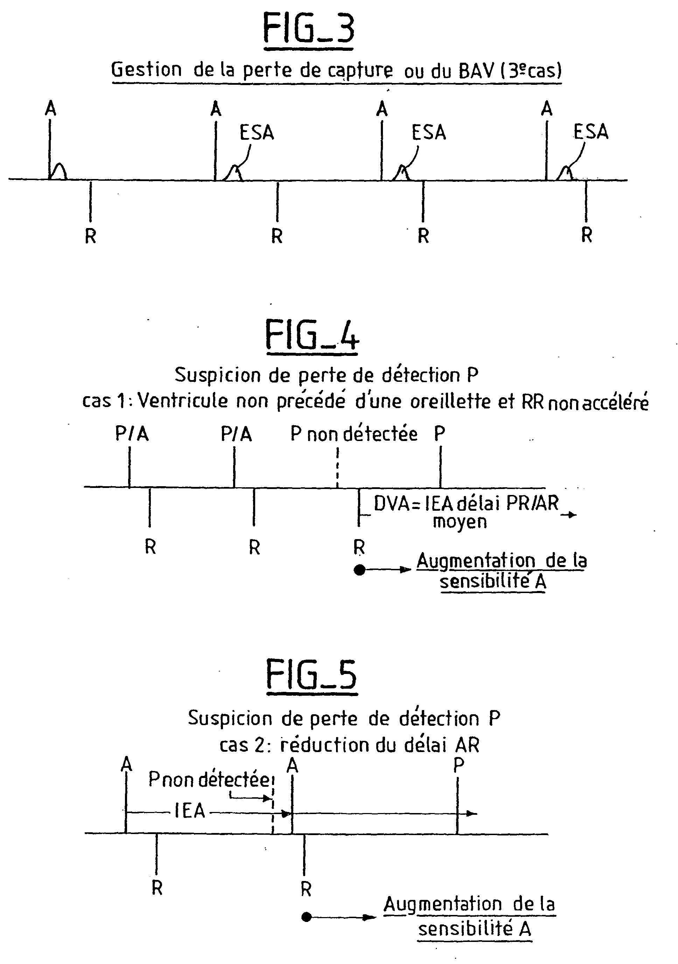

*Extrasystole ventriculaire (ESV) :* détection ventriculaire précédée d'une détection ou d'une stimulation ventriculaire, avec un intervalle de couplage (intervalle R-R ou intervalle V-R) inférieur ou égal à une valeur paramétrable du PP moyen, par exemple inférieur ou égal à 75 % du PP moyen.
Pour de plus amples détails sur la détection et le traitement des extrasystoles, on pourra se référer au EP-A-0 550 342 (Ela Médical), qui décrit un algorithme de détection et de traitement des ESV par une stimulation asynchrone de l'oreillette et une stimulation contrôlée du ventricule.
Pour la mise en oeuvre de l'invention, un certain nombre de fonctions, si elles sont présentes, sont maintenues telles quelles : ainsi, les algorithmes de stimulation, de repli *(fallback)* et de prévention des tachycardies réentrantes électroniques (TRE ou PMT, *Pacemaker-Mediated Tachycardia)* sont maintenus, de même que ceux permettant de calculer et d'appliquer des périodes PRAPA et de protection contre une conduction rétrograde en cas de suspicion d'ESV.
On va maintenant exposer la manière dont, de façon caractéristique de l'invention, le dispositif gère les pertes de capture auriculaire (ou les blocs atrio-ventriculaires (BAV)), ainsi que celles dont il gère les pertes de détection auriculaire.
On va tout d'abord décrire la gestion de la perte de capture, correspondant aux situations illustrées figures 1 à 3.
Le *premier cas de gestion de perte de capture auriculaire* correspond à la situation illustrée sur le chronogramme de la figure 1.
Ce cas est celui d'une absence d'activité ventriculaire après stimulation auriculaire (ce cas étant particulier à un dispositif équipé d'un dispositif à commutation automatique de mode).
Le dispositif suspecte alors une perte de capture auriculaire, et prend les actions suivantes :
- il applique une contre-stimulation auriculaire, si cette fonction a été activée par le praticien (le délai séparant la contre-stimulation de la précédente stimulation auriculaire est paramétrable).
- l'énergie de la stimulation auriculaire suivante, ou de la contre-stimulation, est augmentée. La valeur de l'énergie appliquée est paramétrable et peut être soit l'énergie maximale permise par le dispositif, soit une énergie correspondant à un pas au dessus de l'énergie courante.
Deux possibilités se présentent alors :
. si l'activité ventriculaire est restaurée (branche inférieure du chronogramme de la figure 1), alors le dispositif retrouve un comportement normal, sans DAV, mais avec une énergie de stimulation augmentée pour compenser le risque de perte de capture.
. dans le cas contraire (branche supérieure du chronogramme de la figure 1), si le nombre N1 autorisé de cycles sans activité ventriculaire est atteint, alors tout événement auriculaire non extrasystolique démarre un DAV, et ce pendant N2 cycles, ou bien jusqu'à la survenue d'une détection R non extrasystolique.
Le *deuxième cas de gestion de perte de capture auriculaire* correspond à la situation illustrée sur le chronogramme de la figure 2.
Ce cas est celui de l'allongement du délai de conduction atrio-ventriculaire sur un nombre donné N3 de cycles, ce nombre étant programmable, par exemple N3 = 3 cycles :
- si l'activité auriculaire est systématiquement une activité stimulée, le dispositif suspecte d'abord une perte de capture auriculaire. Dans ce cas, l'énergie de la stimulation suivante est augmentée, à la valeur paramétrée (énergie maximale ou bien une énergie correspondant à un pas au dessus de l'énergie courante), puis :
   . si le délai de conduction atrio-ventriculaire normal est restauré (branche supérieure du chronogramme de la figure 2), le dispositif retrouve le fonctionnement initial AAI, sans DAV, avec une énergie de stimulation augmentée.
   . dans le cas contraire (branche inférieure du chronogramme de la figure 5), l'énergie de stimulation auriculaire initiale est restaurée et l'événement auriculaire non extrasystolique suivant démarre un DAV, et ceci pendant N2 cycles successifs, ou bien jusque la survenue d'une détection R non extrasystolique.
- dans le cas où l'allongement du délai de conduction atrio-ventriculaire était également observé après détection auriculaire, le dispositif peut suspecter d'emblée un BAV et, au terme de N4 cycles, démarrer un DAV, et ceci pendant N2 cycles successifs ou jusque la survenue d'une détection R non extrasystolique.
Le *troisième cas de gestion de perte de capture auriculaire* correspond à la situation illustrée sur le chronogramme de la figure 3.
Ce cas est celui d'une séquence dans laquelle une stimulation A est suivie de la détection d'une onde P, elle-même suivie de la détection d'une onde R spontanée, cette même séquence se répétant sur un nombre programmable donné N4 de cycles, par exemple N4 = 3 cycles.
Cette onde P, décalée, recueillie par le dispositif est considérée par ce dernier comme une ESA.
Le dispositif suspecte alors une perte de capture auriculaire et augmente l'énergie de la stimulation suivante à la valeur paramétrée (énergie maximale ou bien énergie correspondant à un pas au-dessus de l'énergie courante).
Le dispositif dispose également d'une fonctionnalité qui lui permet de restaurer l'énergie de stimulation initiale en cas d'augmentation temporaire.
Toutes les 24 heures, l'énergie de stimulation est abaissée d'un pas. Néanmoins, si une augmentation de l'énergie se produit pendant 3 jours consécutifs, cette "réversibilité" est inhibée.
On va maintenant décrire la manière dont le dispositif de l'invention gère une perte de détection auriculaire.
Cette perte de détection auriculaire correspond aux situations illustrées figures 4 et 5.
Le *premier cas de gestion de perte de détection auriculaire,* illustré figure 4, est celui de la détection d'un événement ventriculaire de type ESV, qui peut laisser supposer une perte de détection auriculaire si son intervalle de couplage est supérieur à une valeur de seuil programmée.
Dans ce cas, la sensibilité auriculaire est augmentée (par exemple diminuée d'un pas) jusqu'à retour d'une détection auriculaire normale, ou retour à une stimulation auriculaire avec un délai normal (c'est-à-dire en l'absence d'accélération du rythme ventriculaire entre détections R successives) entre stimulation auriculaire et détection ventriculaire.
Le *deuxième cas de gestion de perte de détection auriculaire* correspond à la situation illustrée sur le chronogramme de la figure 5.
Si le délai entre stimulation auriculaire et détection ventriculaire diminue d'une quantité supérieure à une durée paramétrable, par exemple de 47 ms cycle à cycle, ou par rapport à un délai défini comme normal, le dispositif suspecte une perte de détection auriculaire et augmente la sensibilité auriculaire au cycle suivant (par exemple en diminuant d'un pas la sensibilité) jusqu'au retour d'un délai normal entre stimulation auriculaire et détection ventriculaire.
Un *troisième cas de gestion de perte de détection auriculaire* est celui du passage d'une détection P à une stimulation A si le délai A-R est inférieur au délai P-R d'une durée paramétrable (par exemple 63 ms) ; le dispositif suspecte alors également une perte de détection, et augmente la sensibilité au cycle suivant.
De la même manière que pour l'énergie de stimulation, toutes les 24 heures, le dispositif diminue la sensibilité pour permettre un retour à la valeur initiale. Néanmoins, si une augmentation de la sensibilité se produit pendant 3 jours consécutifs, cette réversibilité est inhibée.

## Revendications

1. Un dispositif médical implantable actif, notamment un stimulateur cardiaque, défibrillateur et/ou cardioverteur du type double chambre, comprenant :
- des moyens de détection d'événements auriculaires et ventriculaires spontanés.
- des moyens de stimulation ventriculaire et auriculaire, et
- des moyens de suspicion de perte de détection et/ou de capture auriculaire, opérant par analyse de la séquence des stimulations et détections ventriculaire et auriculaire, et
- des moyens de commutation automatique de mode de fonctionnement,
dispositif **caractérisé en ce que** lesdits moyens de suspicion de perte de détection et/ou de capture auriculaire sont des moyens aptes à détecter :
- l'absence d'activité ventriculaire post-stimulatlon auriculaire, et/ou
- l'allongement, au-delà d'une limite donnée, du délai de conduction atrio-ventriculaire sur un nombre prédéterminé de cycles cardiaques successifs, et/ou
- la survenue d'une détection auriculaire consécutive à une stimulation auriculaire sur un nombre prédéterminé de cycles cardiaques successifs, et/ou
- la détection d'une extrasystole ventriculaire, et/ou
- la diminution, en deçà d'une limite donnée, du délai entre stimulation auriculaire et détection ventriculaire, et/ou
- le passage d'une détection auriculaire à une stimulation auriculaire avec diminution concomitante, en deçà d'une limite donnée, du délai entre événement auriculaire et détection ventriculaire,
de manière à éviter un basculement inapproprié en DDD du mode de fonctionnement

2. Le dispositif de la revendication 1, dans lequel lesdits moyens de suspicion de perte de détection et/ou de capture auriculaire sont aptes à commander une contre-stimulation auriculaire d'énergie accrue dans le cas de ladite absence d'activité ventriculaire post-stimulation auriculaire.

3. Le dispositif de la revendication 1, dans lequel lesdits moyens de suspicion de perte de détection et/ou de capture auriculaire sont aptes à commander une augmentation de l'énergie de stimulation auriculaire sur les cycles suivants dans le cas de ladite absence d'activité ventriculaire post-stimulation auriculaire.

4. Le dispositif de la revendication 3, dans lequel lesdits moyens de suspicion de perte de détection et/ou de capture auriculaire sont également aptes à restaurer l'énergie de stimulation auriculaire à sa valeur antérieure en cas de persistance dudit allongement du délai de conduction atrio-ventriculaire.

5. Le dispositif de la revendications 3, dans lequel lesdits moyens de suspicion de perte de détection et/ou de capture auriculaire sont également aptes à opérer à intervalles périodiques un réajustement, à une valeur inférieure, du niveau de l'énergie de stimulation en cas de disparition dudit allongement du délai de conduction atrio-ventriculaire.

6. Le dispositif de la revendications 5, dans lequel lesdits moyens de suspicion de perte de détection et/ou de capture auriculaire sont également aptes à inhiber ledit réajustement en cas de détection d'une augmentation de ladite énergie de stimulation sur un nombre prédéterminé desdits intervalles périodiques consécutifs.

7. Le dispositif de la revendication 1, dans lequel lesdits moyens de suspicion de perte de détection et/ou de capture auriculaire sont aptes à commander une augmentation de l'énergie de stimulation auriculaire sur les cycles suivants dans le cas de ladite survenue de détection auriculaire consécutive à une stimulation auriculaire sur un nombre prédéterminé de cycles cardiaques successifs.

8. Le dispositif de la revendication 7, dans lequel lesdits moyens de suspicion de perte de détection et/ou de capture auriculaire sont également aptes à restaurer l'énergie de stimulation auriculaire à sa valeur antérieure en cas de persistance de ladite survenue de détection auriculaire consécutive à une stimulation auriculaire.

9. Le dispositif de la revendications 8, dans lequel lesdits moyens de suspicion de perte de détection et/ou de capture auriculaire sont également aptes à opérer à intervalles périodiques un réajustement, à une valeur inférieure, du niveau de l'énergie de stimulation en cas de disparition de ladite survenue de détection auriculaire consécutive à une stimulation auriculaire sur un nombre prédéterminé de cycles cardiaques successifs.

10. Le dispositif de la revendications 9, dans lequel lesdits moyens de suspicion de perte de détection et/ou de capture auriculaire sont également aptes à inhiber ledit réajustement en cas de détection d'une augmentation de ladite énergie de stimulation sur un nombre prédéterminé desdits intervalles périodiques consécutifs.

11. Le dispositif de la revendication 1, dans lequel lesdits moyens de suspicion de perte de détection et/ou de capture auriculaire sont aptes à commander une augmentation de la sensibilité des moyens de détection auriculaire.

12. Le dispositif de la revendication 11, dans lequel lesdits moyens de suspicion de perte de détection et/ou de capture auriculaire sont également aptes à restaurer la sensibilité auriculaire à sa valeur antérieure en cas de retour à une stimulation auriculaire induisant un délai non pathologique entre stimulation auriculaire et détection ventriculaire.

## Claims

1. An active implantable medical device, in particular a cardiac pacemaker, defibrillator and/or cardioverter of the double-chamber type, comprising:
- means for detecting spontaneous atrial and ventricular events;
- means for ventricular and atrial stimulation; and
- means for suspecting a loss of atrial detection and/or capture, operating by analyzing the sequence of the ventricular and atrial stimulations and detections; and
- means for automatically switching the operating mode,
said device being **characterised in that** said means for suspecting a loss of atrial detection and/or capture are means adapted to detect:
- the absence of a post-atrial stimulation ventricular activity, and/or
- the lengthening, beyond a given limit, of the atrio-ventricular conduction delay over a predetermined number of successive cardiac cycles, and/or
- the occurrence of an atrial detection consecutive to an atrial stimulation over a predetermined number of successive cardiac cycles, and/or
- the detection of a ventricular extrasystole, and/or
- the reduction, below a given limit, of the delay between atrial stimulation and ventricular detection, and/or
- the passage from an atrial detection to an atrial stimulation with a concomitant reduction, below a given limit, of the delay between atrial event and ventricular detection,
whereby avoiding an inappropriate switching of the operation mode to DDD.

2. The device of claim 1, wherein said means for suspecting a loss of atrial detection and/or capture are adapted to control an atrial counter-stimulation with an increased energy in the event of said absence of post-atrial stimulation ventricular activity.

3. The device of claim 1, wherein said means for suspecting a loss of atrial detection and/or capture are adapted to control an increase in the atrial stimulation energy over the following cycles in the event of said absence of post-atrial stimulation ventricular activity.

4. The device of claim 3, wherein said means for suspecting a loss of atrial detection and/or capture are further adapted to restore the atrial stimulation energy to its prior value in the event of a persistence of said lengthening of the atrio-ventricular conduction delay.

5. The device of claim 3, wherein said means for suspecting a loss of atrial detection and/or capture are further adapted to operate at periodic intervals a readjustment, to a lower value, of the stimulation energy level in the event of a disappearance of said lengthening of the atrio-ventricular conduction delay.

6. The device of claim 5, wherein said means for suspecting a loss of atrial detection and/or capture are further adapted to inhibit said readjustment in the event of an increase in said stimulation energy over a predetermined number of said consecutive periodic intervals.

7. The device of claim 1, wherein said means for suspecting a loss of atrial detection and/or capture are adapted to control an increase of the atrial stimulation energy over the following cycles in the event of said occurrence of an atrial detection consecutive to an atrial stimulation over a predetermined number of successive cardiac cycles.

8. The device of claim 7, wherein said means for suspecting a loss of atrial detection and/or capture are further adapted to restore the atrial stimulation energy to its prior value in the event of a persistence of said occurrence of an atrial detection consecutive to an atrial stimulation.

9. The device of claim 8, wherein said means for suspecting a loss of atrial detection and/or capture are further adapted to operate at periodic intervals a readjustment, to a lower value, of the energy stimulation level in the event of a disappearance of said occurrence of an atrial detection consecutive to an atrial stimulation over a predetermined number of successive cardiac cycles.

10. The device of claim 9, wherein said means for suspecting a loss of atrial detection and/or capture are further adapted to inhibit said readjustment in the event of a detection of an increase in said stimulation energy over a predetermined number of said consecutive periodic intervals.

11. The device of claim 1, wherein said means for suspecting a loss of atrial detection and/or capture are adapted to control an increase in the sensitivity of the atrial detection means.

12. The device of claim 11, wherein said means for suspecting a loss of atrial detection and/or capture are further adapted to restore the atrial sensitivity to its prior value atrial in the event of a return to an atrial stimulation inducing a non-pathological delay between atrial stimulation and ventricular detection.

## Patentansprüche

1. Ein aktive medizinische implantierbare Vorrichtung, insbesondere ein Herzschrittmacher, Defibrillator und/oder Kardioverter vom Typ Doppelkammer, einschließend:
- Mittel zur Feststellung von aurikulären und ventrikulären plötzlichen Ereignissen,
- Mittel zur ventrikulären und aurikulären Stimulierung, und
- Mittel zur Vermutung von aurikulären Feststellungsverlust und/oder Erfassungsverlust, welche durch Analyse der ventrikulären und aurikulären Abfolge der Stimulation und Feststellung wirken,
- Mittel zum automatischen Umschalten der Funktionsweise,
- eine Vorrichtung, **dadurch gekennzeichnet, dass** die Mittel zur Vermutung der aurikulären Feststellung und/oder Erfassung Mittel sind, welche geeignet sind, um festzustellen:
- das Fehlen von aurikulärer Nachstimulation, ventrikulärer Aktivität, und/oder die Verlängerung über eine vorgegebene Grenze hinaus der Verzögerung der atrioventrikulären Leitung auf einer vorbestimmten Anzahl von aufeinanderfolgenden Herzzyklen, und/oder
- das Ereignis einer plötzlichen aurikulären Feststellung auf eine aurikuläre Stimulation auf einer vorherbestimmten Anzahl von auf einanderfolgenden Herzzyklen, und/oder
- das Feststellen einer ventrikulären Extrasystole, und/oder
- die Verringerung unter einem gegebenem Grenzwert, der Verzögerungen zwischen der aurikulären Stimulation und der ventrikulären Feststellung, und/oder
- der Übergang von einer aurikulären Feststellung zu einer aurikulären Stimulation mit Begleitverkleinerung, unter einem vorgegebenen Grenzwert, der Verzögerung zwischen aurikulärem Ereignis und ventrikulärer Feststellung, in der Weise, um unsachgemäßes Umschwenken zu DDD der Funktionsweise zu verhindern.

2. Die Vorrichtung aus Anspruch 1, in welcher die Mittel zur Vermutung des Verlusts der Feststellung und/oder des aurikulären Erfassens geeignet sind, um eine aurikuläre Gegenstimulation von verstärkter Leistung, in dem Fall des Fehlens von aurikulärer Poststimulation, ventrikulärer Aktivität zu regeln.

3. Die Vorrichtung des Anspruchs 1, in welcher die Mittel zur Vermutung des Verlusts von Feststellung und/oder aurikulärer Erfassung geeignet sind, um eine Erhöhung der aurikulären Stimulationsenergie über die nachfolgenden Zyklen, in dem Fall des Fehlens von aurikulärer Poststimulation ventrikulärer Aktivität zu regeln.

4. Die Vorrichtung aus Anspruch 3, in welcher die Mittel zur Vermutung des Verlusts von aurikulärer Feststellung und/oder Erfassung auch geeignet sind, um die aurikuläre Stimulationsenergie auf ihren vorherigen Wert, im Falle einer Andauer der Verlängerung der atrioventrikulären Leitungsverzögerung, wieder herzustellen.

5. Vorrichtung des Anspruchs 3, in welcher die Mittel zur Vermutung von Verlust der aurikulären Feststellung und/oder Erfassung auch geeignet sind, um in periodischen Intervallen Wiederanpassung des Leistungspegels der Stimulation auf einen niedrigeren Wert, im Falle des Verschwindens der Verlängerung der atrioventrikulären Leitungsverzögerung, zu bewirken.

6. Die Vorrichtung des Anspruchs 5, in welcher die Mittel zur Vermutung des Verlusts von aurikulärer Feststellung und/oder Erfassung auch dazu geeignet sind, um die Wiederanpassung im Falle einer Feststellung einer Erhöhung der Stimulationsleistung über eine vorbestimmte Anzahl der aufeinanderfolgenden periodischen Intervalle, zu hemmen.

7. Die Vorrichtung des Anspruchs 1, in welchem die Mittel zur Vermutung des Verlusts der aurikulären Feststellung und/oder Erfassung dazu geeignet sind, eine Erhöhung der aurikulären Stimulationsenergie, in dem Fall eines plötzlichen Ereignisses der aurikulären Feststellung, folgend auf eine aurikuläre Stimulation über eine vorbestimmte Anzahl von aufeinanderfolgenden Herzzyklen, über die folgenden Zyklen zu steuern.

8. Die Vorrichtung des Anspruchs 7, in welchem die Mittel zur Vermutung des Verlusts von aurikulärer Feststellung und/oder Erfassung auch dazu geeignet sind, um die aurikuläre Stimulationsenergie auf ihren vorherigen Wert, im Falle der Andauer des plötzlichen Ereignisses der aurikulären Feststellung auf eine aurikuläre Stimulation folgend, wiederherzustellen.

9. Die Vorrichtung des Anspruchs 8, in welcher die Mittel zur Vermutung des Verlusts von aurikulärer Feststellung und/oder Erfassung auch dazu geeignet sind, um eine Wiederanpassung des Leistungspegels der Stimulation, im Falle des Verschwindens des plötzlichen Ereignisses der aurikulärcn Feststellung infolge einer aurikulären Stimulation über eine vorbestimmte Anzahl von aufeinanderfolgenden Herzzyklen, in periodischen Intervallen zu bewirken.

10. Die Vorrichtung des Anspruchs 9, in welcher die Mittel zur Vermutung des Verlusts von aurikulärer Feststellung und/oder Erfassung auch dazu geeignet sind, um die Wiederanpassung, im Falle der Feststellung einer Erhöhung der Stimulationsenergie über eine vorbestimmte Anzahl der auf einanderfolgenden periodischen Intervalle, zu hemmen.

11. Die Vorrichtung des Anspruchs 1, in welcher die Mittel zur Vermutung des Verlusts von aurikulärer Feststellung und/oder Erfassung dazu geeignet sind, eine Erhöhung der Empfindlichkeit der aurikulären Feststellungsvorrichtung zu erwirken.

12. Die Vorrichtung aus Anspruch 11, in welcher die Mittel zur Vermutung des Verlusts von aurikulärer Feststellung und/oder Erfassung auch dazu geeiget sind, die aurikuläre Empfindlichkeit zu ihrem vorherigen Wert, im Falle der Rückkehr zu einer aurikulären Stimulation, wobei sie eine nichtpathologische Verzögerung zwischen der aurikulären Stimulation und der ventrikulären Feststellung einführt, wiederherzustellen.
